# FASCICULE DE BREVET EUROPEEN

(11) **EP 0 764 441 B1**
(45) Date de publication et mention de la délivrance du brevet: **04.04.2001**
(21) Numéro de dépôt: 96401996.2
(22) Date de dépôt: 19.09.1996
(51) Int. Cl.: A61K 9/70, A61K 7/00

(54) **Patch cosmétique ou dermo-pharmaceutique contenant dans une matrice polymérique anhydre au moins un composé actif, en particulier instable en milieu oxydant, et au moins un agent hydro-absorbant**
Kosmetisches oder dermopharmazeutisches Pflaster, enthaltend mindestens einen Wirkstoff, insbesondere einen oxidationslabilen, und mindestens einen Hydroabsorber in einer wasserfreien Polymermatrix
Cosmetic or dermopharmaceutical patch containing at least one active agent, particularly an unstable one in an oxidative medium, and at least one hydroabsorbent in an anhydrous polymeric matrix

(30) Priorité: 20.09.1995 FR 9511030
(43) Date de publication de la demande: 26.03.1997
(73) Titulaire: L'OREAL, 75008 Paris (FR)
(72) Inventeur: Gueret, Jean-Louis, 75018 Paris (FR)
(74) Mandataire: Stalla-Bourdillon, Bernard

(56) Documents cités:
- EP-A- 0 137 278
- EP-A- 0 190 814
- EP-A- 0 196 769
- EP-A- 0 309 309
- EP-A- 0 379 933
- EP-A- 0 412 869
- WO-A-93/19789

## Description

La présente invention concerne un patch permettant la libération contrôlée sur la surface de la peau d'au moins un composé, cosmétiquement ou dermo-pharmaceutiquement actif, notamment d'un composé instable en milieu oxydant.

Selon la présente description, on désigne sous le terme de patch une structure composite sous forme de couches qui, par application sur la peau, assure la libération d'un produit actif à sa surface.

Lorsque le composé actif est instable en milieu oxydant, il s'agit notamment d'un composé capable de subir une dégradation après un temps de contact suffisant avec un milieu oxydant, par exemple par hydrolyse après quelques heures en milieu aqueux.

Il est déjà connu d'utiliser des patchs permettant, par transdermie, de faire pénétrer des composés actifs.

De tels patchs présentent en général une structure comportant plusieurs couches successives dans l'ordre suivant: une première couche, dite couche support, généralement occlusive, c'est-à-dire constituée d'une matière imperméable au composé actif de façon à empêcher l'évaporation de ce dernier et faciliter la transdermie ; une seconde couche, dite couche réservoir, fixée sur la couche support et contenant le composé actif, cette couche pouvant venir directement au contact de la peau ; éventuellement, pour faciliter la fixation du patch sur la peau, une couche d'une matière adhésive appliquée à la surface de la couche réservoir et perméable au composé actif ; enfin, une couche détachable de protection, recouvrant de façon hermétique la couche réservoir, de façon à la protéger de toute contamination extérieure pendant le temps de stockage préalable à l'utilisation du patch.

On connaît en particulier selon EP 412 869 une structure de patch constituée d'une couche support occlusive et comprenant, fixée sur cette dernière, une couche réservoir en une matrice d'un polymère de silicone incluant, à l'état dispersé, des particules de gel aqueux contenant au moins un composé cosmétiquement ou pharmaceutiquement actif. Si cette forme de patch est plus particulièrement appropriée pour la délivrance de composés actifs peu sensibles aux phénomènes d'oxydation, elle est par contre d'une utilité très médiocre pour les composés actifs instables en milieu oxydant.

En effet, dans ce dernier cas, le composé actif étant présent dans les particules de gel aqueux, celui-ci se dégrade rapidement par oxydation au contact du milieu.

Il a également été décrit dans la demande EP 190.814 un pansement occlusif essentiellement constitué d'une couche flexible en mousse de polyuréthanne à cellules fermées contenant, dans le réseau de la mousse, environ 5 à 50 % en poids par rapport à la mousse d'au moins un agent dispersible dans l'eau, gonflable dans l'eau et/ou hydro-absorbant et éventuellement en une proportion inférieure à 5 % en poids d'au moins un composé pharmaceutiquement actif.

Ce type de pansement plus particulièrement adapté aux traitements des plaies présente l'inconvénient d'être sous forme de mousse à cellules fermées de telle sorte que la libération du composé actif, lorsqu'il est présent, est lente et ne se produit que lorsque l'exsudat des plaies a pénétré à l'intérieur de la mousse. On comprend de ce fait que selon la demande EP 190.814, l'action principale du pansement lorsqu'il est appliqué sur une plaie, est essentiellement de l'assécher par migration de l'exsudat qui réagit alors avec l'agent dispersible dans l'eau, gonflable dans l'eau et/ou hydro-absorbant.

La présence éventuelle d'un composé actif tel qu'un antibiotique, un antimicrobien ou un antiseptique, est donc secondaire car il n'est pas destiné à diffuser directement sur la plaie, mais à agir sur l'exsudat absorbé par la mousse à cellules fermées.

Il a par ailleurs été décrit dan la demande EP 196.769 un patch transdermique comprenant une couche support, une couche réservoir constituée d'une matrice polymérique solide sous forme de disque dans laquelle est dispersée une quantité appropriée d'un composé pharmaceutique et sur laquelle est appliquée une couche adhésive, celle-ci contenant une quantité efficace d'au moins un agent permettant d'augmenter la transdermie du composé pharmaceutique.

Ce type de patch, s'il permet une meilleure disponibilité du composé pharmaceutiquement actif, est toutefois de réalisation délicate car le composé présent dans la couche adhésive devant permettre d'augmenter la transdermie, doit être choisie en fonction de la nature du composé actif et ce composé qui vient au contact de la peau, doit être tel qu'il ne provoque aucune réaction allergique.

La présente invention permet de remédier aux inconvénients rencontrés dans l'état de la technique lorsque le composé actif est notamment instable en milieu oxydant.

En effet, on a constaté d'une manière tout à fait surprenante et inattendue qu'en dispersant de façon homogène, dans une matrice hydrophobe spécifique, au moins un composé cosmétiquement ou dermo-pharmaceutiquement actif, en particulier instable en milieu oxydant, avec certains agents hydro-absorbants, il était possible d'obtenir un patch comportant une couche réservoir anhydre, mais permettant néanmoins une excellente libération contrôlée du composé actif sans aucune dégradation de ce dernier.

Il s'est en effet avéré qu'après enlèvement de la couche détachable de protection, le patch pouvait être appliqué directement sur la surface de peau à traiter sans qu'il soit impérativement nécessaire, pour obtenir l'effet recherché, d'humidifier soit la partie de la peau à traiter, soit le patch lui-même.

La couche support étant au moins partiellement occlusive provoque, sur la partie de la peau où le patch a été appliqué, une condensation de la transpiration et permet ainsi de l'humidifier de façon suffisante en vue de la libération du composé actif par l'intermédiaire des particules de l'agent hydro-absorbant qui y sont dispersées.

En effet, au contact de l'humidité de la peau (ou éventuellement en présence d'eau appliquée sur la peau ou sur la couche réservoir) les particules de l'agent hydro-absorbant réagissent et libèrent alors progressivement les particules du composé actif.

Ainsi, la présente invention a pour objet un patch cosmétique ou dermo-pharmaceutique pour la libération contrôlée d'au moins un composé cosmétiquement ou dermo-pharmaceutiquement actif sur la peau comportant une couche réservoir, fixée sur une couche support, ladite couche réservoir étant constituée d'une matrice polymérique hydrophobe dans laquelle sont dispersées de façon homogène des particules d'au moins un composé actif, éventuellement instable en milieu oxydant, et des particules d'au moins un agent hydro-absorbant, ladite couche réservoir étant du type compact et anhydre.

Par le terme "compact" on entend selon l'invention, que la couche réservoir est sous forme d'une masse dense ne comportant par conséquent pas d'interstices alvéolaires ou cellulaires contrairement à la couche réservoir selon la demande EP 190.814 qui se présente sous forme d'une mousse de polyuréthanne ou plus précisément de polyuréthanne expansé.

Les particules du composé actif et de l'agent hydro-absorbant étant à l'état dispersé et sous forme homogène dans la couche réservoir, une partie au moins desdites particules se trouvent être présentes à la surface de la couche réservoir venant au contact direct de la peau ce qui permet, après enlèvement de la couche détachable de protection, une action de libération du composé actif particulièrement efficace et rapide ce qui ne pouvait être obtenu selon l'état de la technique.

Dans les patchs selon la présente invention, la matrice polymérique hydrophobe est par exemple à base d'un polymère de silicone ou d'un polyuréthanne du type polyester polyuréthanne ou polyéther polyuréthanne.

Lorsque la matrice polymérique est à base d'un polymère de silicone, le prépolymère de silicone est de préférence choisi parmi les organopolysiloxanes linéaires substitués sur l'atome de silicium par des divers groupes, les atomes de silicium terminaux étant trisubstitués. De tels organopolysiloxanes sont décrits notamment dans les brevets US-2541137, 2723966, 2863846, 2890188, 2927907, 3002951 et 3035016.

On préfère en particulier en tant que prépolymère de silicone les polydiméthylsiloxanes de formule : 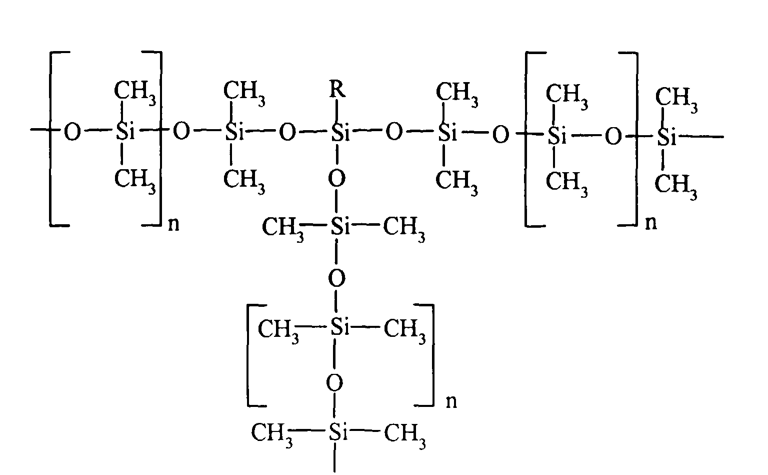 dans laquelle :
R représente un groupe alkyle ou alkoxy contenant de 1 à 7 atomes de carbone, un groupe vinyle ou phényle, et dans laquelle n est compris entre environ 100 et 5.000.

Le prépolymère de silicone utilisé est réticulable de préférence à des températures modérées telles que la température ambiante, en utilisant un catalyseur de réticulation biologiquement acceptable dans la matrice polymérique résultante et qui est compatible avec le composé actif dispersé dans cette dernière.

Par catalyseur de réticulation, on entend, selon la présente invention, l'association d'un agent de réticulation et d'un catalyseur.

Lorsque le prépolymère de silicone comporte des groupes hydroxy, tels que des groupes hydroxy terminaux, on peut citer, comme agent de réticulation, le tétrapropoxysilane [Si-(O-CH₂-CH₂-CH₃)₄] en association avec un catalyseur à base d'étain.

Lorsque le prépolymère de silicone comporte des groupes vinyliques, on peut réticuler ce dernier en présence d'un polymère de diméthyl-silicone en association avec un catalyseur tel qu'un catalyseur à base de platine.

Parmi les prépolymères de silicone particulièrement préférés selon l'invention, on peut citer ceux connus sous les dénominations de SILASTIC 382®, Q7-4635®, Q7-4650®, Q7-4735®, Q7-4750®, Q7-4765®, MDX 4-4210"® et DC 3.6486® commercialisés par la Société DOW CORNING.

Lorsque la matrice polymérique est à base d'un polyuréthanne, celle-ci est obtenue à partir d'un prépolymère du type polyester-polyol ou polyéther-polyol connu dans l'état de la technique. Parmi les polyesters-polyols on peut citer ceux obtenus par réaction d'alcools bi- ou trifonctionnels sur des acides tels que l'acide adipique, l'acide téréphtalique et de façon plus générale, tous autres acides plurifonctionnels. Parmi les polyéthers-polyols on peut citer ceux obtenus par alcoylation en faisant réagir des diols tels que l'éthylène glycol ou le propylène glycol ou des polyols tels que le triméthylolpropane, le glycérol, le pentaérythritol, le sorbitol etc. avec des oxydes tels que l'oxyde d'éthylène, l'oxyde de propylène ou leur mélange.

L'agent de polyaddition pour la formation des polyuréthannes est un isocyanate ou polyisocyanate, notamment le toluène diisocyanate, le diphényl-méthane-4,4' diisocyanate, le naphtalène-1,5 diisocyanate ou l'isophorone diisocyanate.

Le catalyseur de réticulation ou l'agent de polyaddition est de préférence utilisé en une quantité telle que la réticulation ou la polyaddition ne soit pas complète, ceci de façon à ce que la couche réservoir présente en elle-même un caractère auto-adhésif satisfaisant pour ainsi avantageusement éviter que la couche support ne soit ultérieurement revêtue d'une couche adhésive.

On remarquera, toutefois que les patchs selon l'invention peuvent ne pas nécessairement adhérer à la surface de la peau.

En effet, en fonction du temps d'application, ils peuvent être soit maintenus en place par le sujet traité, soit utilisés pour un massage d'une zone déterminée de peau à traiter.

Dans les patchs selon la présente invention, le composé cosmétiquement ou dermo-pharmaceutiquement actif, peut être par exemple de la vitamine C, de la vitamine A ou rétinol, de la vitamine F (acides gras essentiels), des enzymes, des antibiotiques tels que le phosphate de clindamycine.

Parmi les agents hydro-absorbants pouvant être présents dans la matrice polymérique hydrophobe à l'état dispersé, on peut citer, de préférence, les polyacrylates réticulés superabsorbants à fort taux de gonflement dans l'eau, tels que ceux commercialisés par la Société NORSOLOR sous la dénomination "Aquakeep"® ; l'alcool polyvinylique ; les polymères carboxyvinyliques tels que ceux commercialisés par la Société GOODRICH sous les dénominations de "Carbopol"® ; les dérivés semi-synthétiques de la cellulose tels que la carboxyméthylcellulose ; les substances naturelles telles que les amidons, les gommes naturelles (gomme de guar, gomme arabique, gomme adragante), la caséine, les phytocolloïdes (carragénates, alginates, agar-agar), les fibres de coton et la gélatine.

On préfère tout particulièrement utiliser selon l'invention les polyacrylates réticulés superabsorbants dont la présence à l'état dispersé dans la matrice polymérique hydrophobe favorise, après hydratation, un meilleur contact avec les particules de composé actif.

L'agent hydro-absorbant peut être également selon une forme de réalisation particulière des particules de poudre d'émulsions lyophilisées ou atomisées, ces particules pouvant contenir au moins une substance active.

De préférence, le composé cosmétiquement ou dermo-pharmaceutiquement actif est présent en une proportion comprise entre environ 0,2 et 48 % en poids et l'agent hydro-absorbant en une proportion comprise entre environ 0,1 et 30 % en poids, de préférence entre 0,5 et 10 %, par rapport au poids total de la couche réservoir sous réserve que la proportion du mélange du composé actif et de l'agent hydro-absorbant soit comprise de préférence entre environ 15 et 60 % en poids par rapport au poids total de la couche réservoir.

Le composé cosmétiquement ou dermo-pharmaceutiquement actif, de même que l'agent hydro-absorbant, sont présents à l'état dispersé dans la matrice polymérique sous forme de particules de taille moyenne comprise entre environ 0,2 µm et 1,5 mm, lesdites particules étant dispersées de manière homogène et aléatoire dans la matrice polymérique, constituant la couche réservoir.

Du fait de cette dispersion aléatoire dans la matrice polymérique, les patchs selon la présente invention permettent d'incorporer, à l'état dispersé dans la matrice polymérique, différents composés actifs non seulement instables en eux-mêmes mais également incompatibles entre eux.

Cette forme de réalisation est particulièrement avantageuse lorsqu'au moins deux composés actifs incompatibles entre eux sont dispersés au sein de la matrice polymérique mais présentent, lors de leur libération, au contact de la peau, un effet de synergie.

Tel est le cas par exemple lorsqu'on disperse dans la matrice polymérique des particules de vitamine C associées à des particules d'enzymes.

Par ailleurs, la matrice polymérique peut en outre contenir des ingrédients cosmétiques ou dermo-pharmaceutiques additionnels tels que des huiles émollientes, des composants à effet tenseur tels que des poudres de protéines de soja ou de blé.

En outre, la matrice polymérique peut avantageusement contenir un agent effervescent tel que du bicarbonate ou du carbonate de sodium afin de favoriser par effervescence l'action du composé actif libéré à partir de la couche réservoir.

Enfin, afin de renforcer la résistance à l'allongement de la matrice polymérique, la couche réservoir peut comporter une trame, constituée par exemple d'une feuille d'un matériau plastique perforée, d'une feuille d'un non-tissé perforée, ou d'un filet, le non-tissé ou le filet étant constitué de fibres naturelles ou synthétiques telles que du polyamide comme décrit dans le brevet français n° 92 05623 (FR-A-2620914).

La couche support ou occlusive des patchs selon la présente invention peut être constituée de tout matériau approprié imperméable au composé actif contenu dans la couche réservoir adjacente.

La couche support a non seulement pour fonction de supporter la couche réservoir mais également de servir de revêtement protecteur de celle-ci.

Elle peut être de même dimension que la couche réservoir ou de dimension plus grande de telle sorte qu'elle s'étende au-delà la périphérie de la couche réservoir et vers l'extérieur, de manière à ce que la surface qui entoure la couche réservoir puisse éventuellement recevoir des moyens adhésifs.

Parmi les matériaux appropriés pour la couche support, on peut citer des films de polyéthylène haute et basse densité, des films de polypropylène, de polychlorure de vinyle, de polyester tel que le polyphtalate d'éthylène, de copolymères éthylène-acétate de vinyle et de polyuréthanne.

Ces matériaux peuvent également être présents sous forme de stratifié avec au moins une feuille de métal telle qu'une feuille d'aluminium. La couche support peut être de toute épaisseur appropriée qui procurera les fonctions de support et de protection souhaitées. De préférence, l'épaisseur de la couche support est comprise entre environ 0,2 et 1,5 mm.

Les patchs selon la présente invention peuvent être protégés par la présence d'une couche détachable ou pelable de protection adjacente à la couche réservoir et/ou par un conditionnement dans un emballage approprié, notamment imperméable à l'eau et à la vapeur d'eau.

Lorsque la couche réservoir est protégée par une couche détachable de protection, celle-ci est enlevée au moment de l'utilisation. Elle peut être constituée en tout matériau imperméable au composé actif ainsi qu'à tout autre composant présent dans la matrice polymérique. Parmi les matériaux pouvant être utilisés, on peut citer de préférence une feuille de papier siliconée ou une feuille de matériau thermoplastique traitée pour la rendre anti-adhérente, par exemple à l'aide d'un vernis. De préférence, cette couche détachable de protection est constituée de polyéthylène.

De façon connue, les patchs selon la présente invention peuvent être découpés selon un contour approprié correspondant à la zone de surface de peau à traiter, par exemple sous forme de masque pour l'application sur le visage, notamment pour l'application sur les contours des yeux, sur les poches sous les yeux, sur le front, sur le nez (protège-nez solaire). Bien entendu, les patchs selon la présente invention peuvent être découpés sous toute autre forme nécessaire pour une application sur une zone déterminée du corps.

Les patchs ainsi constitués et découpés peuvent être utilisés, après élimination de la couche détachable de protection, sur une surface de peau à traiter, en les appliquant directement sur une peau dont l'eau de transpiration permettra d'obtenir la libération désirée du composé cosmétiquement ou dermo-pharmaceutiquement actif. Ils peuvent également être préalablement trempés dans de l'eau pendant un temps de préférence compris entre environ 5 et 30 secondes, ou être appliqués sur la peau après l'avoir préalablement mouillée par exemple à l'aide d'une éponge.

Il a en effet été constaté de manière surprenante et inattendue que la seule eau de transpiration de la peau permettait avantageusement d'obtenir la libération du composé actif sur la surface de la peau à traiter, à partir de la matrice polymérique hydrophobe le contenant.

Dans les patchs selon la présente invention, la matrice polymérique constituant la couche réservoir est préparée par mélange intime sous agitation du prépolymère de silicone ou de polyuréthanne, du composé cosmétiquement ou dermo-pharmaceutiquement actif et de l'agent hydro-absorbant, tous deux sous forme de particules, ainsi que des composants optionnels mentionnés ci-dessus.

Au mélange ainsi obtenu, on ajoute alors à basse température, en général à température ambiante, soit un catalyseur de réticulation si le prépolymère est un polymère de silicone, soit un isocyanate ou polyisocyanate si le prépolymère est un polyester-polyol ou un polyéther-polyol.

Le mélange est alors introduit dans une trémie et versé sur une feuille de polyéthylène par exemple, constituant la pellicule de protection détachable ou pelable du patch. En aval de la trémie est disposé une racle permettant d'égaliser l'épaisseur de la couche réservoir de la matrice polymérique, celle-ci étant généralement comprise entre 0,1 mm et 12 mm.

On applique ensuite une feuille de trame tel que définie ci-dessus, provenant d'un rouleau, puis avant calandrage, une feuille de la couche support ou occlusive qui peut être également une feuille de polyéthylène elle-même provenant d'un rouleau.

La polymérisation ou polyaddition est de préférence réalisée à température ambiante ceci en vue de ne pas détériorer le ou les composés actifs.

Après calandrage et avant que la polymérisation ou polyaddition ne soit complète, la structure composite obtenue peut être immédiatement découpée aux formes voulues, ce qui permet d'obtenir des bords pincés évitant tout phénomène de coulage.

De façon générale, la polymérisation ou polyaddition est complète après environ 24 heures à température ambiante.

La présente invention a également pour objet l'utilisation d'un patch tel que défini ci-dessus, celle-ci étant caractérisée par le fait que le patch est appliqué à l'état sec et de façon occlusive sur une surface de peau à traiter.

Les patchs selon la présente invention permettent d'obtenir un effet très rapide, après application généralement de l'ordre de 5 à 10 minutes.

L'exemple suivant permet d'illustrer la présente invention.

### EXEMPLE

A 8 g de polyacrylate en poudre ("Aquakeep"® commercialisé par la Société NORSOLOR), on ajoute 2 g de carbonate de sodium et 2 g de vitamine C. On micronise ensuite à la granulométrie désirée puis ajoute 43 g d'organopolysiloxane "DC3.6486"® (commercialisé par la Société DOW CORNING). Sous agitation à 1.500 tours/min., on ajoute 1,7 g de son catalyseur de réticulation "Medical Grade Curing Agent" et on maintient l'agitation pendant quelques minutes.

Le produit ainsi homogénéisé est introduit dans une trémie et est étalé à l'aide d'une racle en une couche de 0,8 mm d'épaisseur sur une feuille de polyéthylène d'une épaisseur de 200 µm. Cette feuille peut être préalablement traitée en surface pour réduire son adhérence. Sur la feuille de polyéthylène ainsi revêtue, on applique une trame constituée par un filet de polyamide ou de polyéthylène comportant des mailles d'une ouverture de 1 mm et d'une épaisseur de 0,3 mm.

On applique ensuite un film de polyéthylène (sans traitement anti-adhérence) de 30 µm d'épaisseur qui constitue la couche support ou occlusive du patch, et on procède au calandrage de l'ensemble. On obtient ainsi un ensemble comportant une couche support occlusive et une couche réservoir auto-adhésive formée d'une matrice en polymère de silicone partiellement réticulé, cet ensemble comprenant en outre, une couche détachable de protection.

A partir de cet ensemble, on peut réaliser par découpe, différentes formes de patch en fonction des utilisations souhaitées.

Les patchs après découpe sont ensuite conditionnés dans des sachets de polyéthylène. Lors de l'utilisation, le patch, après élimination de la couche détachable de protection, est appliqué directement sur le contour d'un oeil, par exemple pendant une période de 7 minutes environ. Après avoir retiré le patch, on constate visuellement que le contour de l'oeil traité à l'aide du patch contenant de la vitamine C, présente de manière sensible un teint plus clair, un aspect plus lisse et plus reposé que celui de l'oeil non traité.

## Revendications

1. Patch cosmétique ou dermo-pharmaceutique pour la libération contrôlée d'au moins un composé cosmétiquement ou dermo-pharmaceutiquement actif sur la peau, comportant une couche réservoir, fixée sur une couche support, caractérisé par le fait que ladite couche réservoir est constituée d'une matrice polymérique hydrophobe dans laquelle sont dispersées de façon homogène des particules du composé actif, éventuellement instable en milieu oxydant, et des particules d'au moins un agent hydro-absorbant, ladite couche réservoir étant du type compact et anhydre.

2. Patch selon la revendication 1, caractérisé par le fait que la matrice polymérique hydrophobe est à base d'un polymère de silicone ou d'un polyuréthanne.

3. Patch selon la revendication 1 ou 2, caractérisé par le fait qu'il est auto-adhésif.

4. Patch selon la revendication 1 ou 2, caractérisé par le fait qu'il est non adhésif.

5. Patch selon l'une quelconque des revendications précédentes, caractérisé par le fait que la matrice polymérique à base de polymère de silicone est obtenue par réticulation d'un organopolysiloxane linéaire substitué sur l'atome de silicium par des groupes choisis parmi un groupe alkyle en C₁-C₆, aryle ou ar(alkyle en C₁-C₂), les atomes de silicium terminaux étant trisubstitués.

6. Patch selon la revendication 5, caractérisé par le fait que l'organopolysiloxane a pour formule générale : 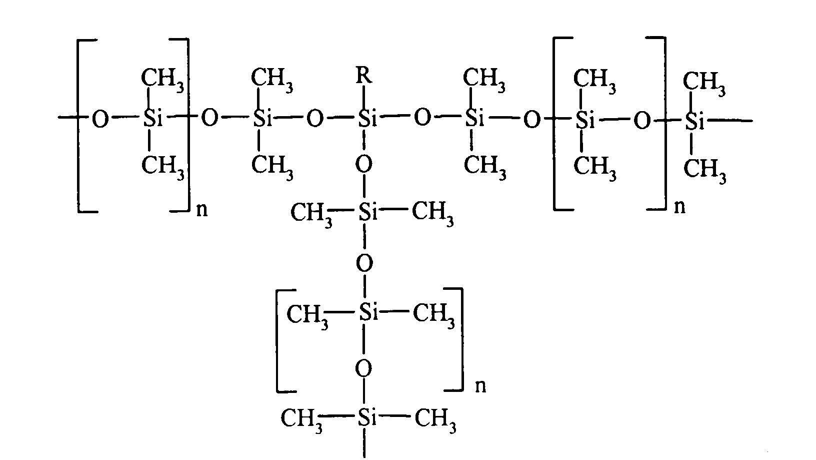 dans laquelle :
R représente un groupe alkyle ou alkoxy contenant de 1 à 7 atomes de carbone, un groupe vinyle ou phényle, et dans laquelle n est compris entre environ 100 et 5.000.

7. Patch selon l'une quelconque des revendications 1 à 4, caractérisé par le fait que la matrice polymérique à base d'un polyuréthanne est obtenue par polyaddition d'un polyester-polyol ou d'un polyéther-polyol en présence d'un isocyanate ou polyisocyanate choisi parmi le toluène diisocyanate, le diphénylméthane-4,4' diisocyanate, le naphtalène-1,5 diisocyanate et l'isophorone diisocyanate.

8. Patch selon l'une quelconque des revendications précédentes, caractérisé par le fait que le composé cosmétiquement ou dermopharmaceutiquement actif, est choisi parmi la vitamine C, la vitamine A, la vitamine F, les enzymes et les antibiotiques.

9. Patch selon l'une quelconque des revendications précédentes, caractérisé par le fait que l'agent hydro-absorbant est choisi parmi les polyacrylates réticulés superabsorbants, l'alcool polyvinylique, les polymères carboxyvinyliques, les dérivés semi-synthétiques de la cellulose, les amidons, les gommes de guar, arabique ou adragante, la caséine, les phytocolloïdes, les fibres de coton et la gélatine.

10. Patch selon l'une quelconque des revendications 1 à 8, caractérisé par le fait que l'agent hydro-absorbant est sous forme de particules d'une poudre d'émulsions lyophilisées ou atomisées contenant éventuellement au moins une substance active.

11. Patch selon l'une quelconque des revendications précédentes, caractérisé par le fait que le composé actif est présent dans la couche réservoir en une proportion comprise entre environ 0,2 et 48 % en poids par rapport au poids total de ladite couche.

12. Patch selon l'une quelconque des revendications 1 à 10, caractérisé par le fait que l'agent hydro-absorbant est présent dans la couche réservoir en une proportion comprise entre environ 0,1 et 30 % en poids par rapport au poids total de ladite couche.

13. Patch selon les revendications 11 et 12, caractérisé par le fait que la proportion du mélange du composé actif et de l'agent absorbant est comprise entre environ 15 et 60 % en poids par rapport au poids total de la couche réservoir.

14. Patch selon l'une quelconque des revendications précédentes, caractérisé par le fait que les particules du composé actif et de l'agent hydro-absorbant ont une taille moyenne comprise entre environ 0,2 µm et 1,5 mm.

15. Patch selon l'une quelconque des revendications précédentes, caractérisé par le fait que la couche réservoir comprend en outre au moins un ingrédient cosmétique ou dermo-pharmaceutique choisi parmi les huiles émollientes, les poudres de protéines de soja ou de blé.

16. Patch selon l'une quelconque des revendications précédentes, caractérisé par le fait que la couche réservoir comprend en outre un agent effervescent choisi parmi le bicarbonate de sodium et le carbonate de sodium.

17. Patch selon l'une quelconque des revendications précédentes, caractérisé par le fait que la couche réservoir comporte une trame choisie parmi une feuille d'un matériau plastique perforée, une feuille perforée d'un non-tissé de fibres naturelles ou synthétiques et un filet en fibres naturelles ou synthétiques.

18. Patch selon l'une quelconque des revendications précédentes, caractérisé par le fait que le fait que l'épaisseur de la couche réservoir est comprise entre environ 0,1 mm et 12 mm.

19. Patch selon l'une quelconque des revendications précédentes, caractérisé par le fait que la couche support est constituée d'un polymère choisi parmi les polyéthylènes haute et basse densité, les polypropylènes, les polychlorure de vinyle, les copolymères d'éthylène et d'acétate de vinyle, les polyesters et les polyuréthannes.

20. Patch selon l'une quelconque des revendications précédentes, caractérisé par le fait que le fait que l'épaisseur de la couche support est comprise entre environ 0,2 mm et 1,5 mm.

21. Utilisation cosmétique d'un patch pour la libération contrôlée, sur la surface de la peau, d'au moins un composé cosmétiquement actif, par application dudit patch sur ladite surface, ledit patch comportant une couche réservoir de type compact et anhydre, fixée sur une couche support, et constituée d'une matrice polymérique hydrophobe dans laquelle sont dispersées de façon homogène des particules du composé actif, éventuellement instable en milieu oxydant, et des particules d'au moins un agent hydro-absorbant, l'utilisation thérapeutique étant exclue.

22. Utilisation en cosmétique d'un patch selon la revendication 21, caractérisée par le fait que ledit patch est appliqué à l'état sec et de façon occlusive sur ladite surface de la peau.

23. Utilisation en cosmétique d'un patch selon la revendication 21, caractérisée par le fait que ledit patch est appliqué après humidification de ladite surface de la peau.

## Claims

1. Cosmetic or dermopharmaceutical patch for the controlled release of at least one compound which is cosmetically or dermopharmaceutically active on the skin, comprising a reservoir layer, fixed to a support layer, characterized in that the said reservoir layer consists of a hydrophobic polymer matrix in which are uniformly dispersed particles of the active compound, which may be unstable in oxidative medium, and particles of at least one water-absorbing agent, the said reservoir layer being of the compact and anhydrous type.

2. Patch according to Claim 1, characterized in that the hydrophobic polymer matrix is based on a silicone polymer or a polyurethane.

3. Patch according to Claim 1 or 2, characterized in that it is self-adhesive.

4. Patch according to Claim 1 or 2, characterized in that it is non-adhesive.

5. Patch according to any one of the preceding claims, characterized in that the polymer matrix based on silicone polymer is obtained by crosslinking a linear polyorganosiloxane substituted on the silicon atom with groups chosen from a C₁-C₆ alkyl group, an aryl group and an ar(C₁-C₂ alkyl) group, the terminal silicon atoms being trisubstituted.

6. Patch according to Claim 5, characterized in that the polyorganosiloxane has the general formula: 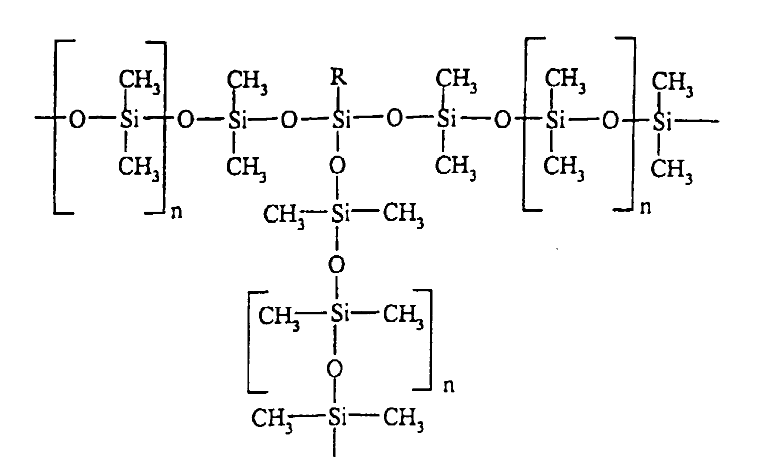 in which:
R represents an alkyl or alkoxy group containing from 1 to 7 carbon atoms, a vinyl group or a phenyl group, and in which n is between about 100 and 5 000.

7. Patch according to any one of Claims 1 to 4,
characterized in that the polymer matrix based on a polyurethane is obtained by polyaddition of a polyesterpolyol or of a polyetherpolyol in the presence of an isocyanate or polyisocyanate chosen from tolylene diisocyanate, 4,4'-diphenylmethane diisocyanate, 1,5-naphthalene diisocyanate and isophorone diisocyanate.

8. Patch according to any one of the preceding claims, characterized in that the compound which is cosmetically or dermopharmaceutically active is chosen from vitamin C, vitamin A, vitamin F, enzymes and antibiotics.

9. Patch according to any one of the preceding claims, characterized in that the water-absorbing agent is chosen from superabsorbent crosslinked polyacrylates, polyvinyl alcohol, carboxyvinyl polymers, semisynthetic cellulose derivatives, starches, guar gum, gum arabic, gum tragacanth, casein, phytocolloids, cotton fibres and gelatin.

10. Patch according to any one of Claims 1 to 8,
characterized in that the water-absorbing agent is in the form of particles of a powder of freeze-dried or atomized emulsions optionally containing at least one active substance.

11. Patch according to any one of the preceding claims, characterized in that the active compound is present in the reservoir layer in a proportion of between about 0.2% and 48% by weight relative to the total weight of the said layer.

12. Patch according to any one of Claims 1 to 10,
characterized in that the water-absorbing agent is present in the reservoir layer in a proportion of between about 0.1% and 30% by weight relative to the total weight of the said layer.

13. Patch according to Claims 11 and 12,
characterised in that the proportion of a mixture of the active compound and of the absorbing agent is between about 15% and 60% by weight relative to the total weight of the reservoir layer.

14. Patch according to any one of the preceding claims, characterized in that the particles of the active compound and of the water-absorbing agent have an average size of between about 0.2 µm and 1.5 mm.

15. Patch according to any one of the preceding claims, characterized in that the reservoir layer also comprises at least one cosmetic or dermopharmaceutical ingredient chosen from emollient oils and soybean or wheat protein powders.

16. Patch according to any one of the preceding claims, characterized in that the reservoir layer also comprises an effervescent agent chosen from sodium bicarbonate and sodium carbonate.

17. Patch according to any one of the preceding claims, characterized in that the reservoir layer comprises a frame chosen from a perforated sheet of a plastic material, a perforated sheet of a nonwoven of natural or synthetic fibres and a net of natural or synthetic fibres.

18. Patch according to any one of the preceding claims, characterized in that the thickness of the reservoir layer is between about 0.1 mm and 12 mm.

19. Patch according 'to any one of the preceding claims, characterized in that the support layer consists of a polymer chosen from high-density and low-density polyethylenes, polypropylenes, polyvinyl chlorides, copolymers of ethylene and of vinyl acetate, polyesters and polyurethanes.

20. Patch according to any one of the preceding claims, characterized in that the thickness of the support layer is between about 0.2 mm and 1.5 mm.

21. Cosmetic use of a patch for the controlled release, onto the surface of the skin, of at least one cosmetically active compound, by applying the said patch to the said surface, the said patch comprising a reservoir layer of compact and anhydrous type, fixed to a support layer, and consisting of a hydrophobic polymer matrix in which are uniformly dispersed particles of the active compound, which may be unstable in oxidative medium, and particles of at least one water-absorbing agent, therapeutic use being excluded.

22. Cosmetic use of a patch according to Claim 21,
characterized in that the said patch is applied occlusively in dry form onto the said surface of the skin.

23. Cosmetic use of a patch according to Claim 21,
characterized in that the said patch is applied after wetting the said surface of the skin.

## Patentansprüche

1. Kosmetisches oder dermo-pharmazeutisches Pflaster für die kontrollierte Freisetzung mindestens einer kosmetisch oder dermo-pharmazeutisch aktiven Verbindung auf der Haut, umfassend eine auf einer Trägerschicht angebrachten Speicherschicht, dadurch gekennzeichnet, dass die Speicherschicht aus einer hydrophoben Polymermatrix besteht, in der in homogener Weise Wirkstoffteilchen, die gegebenenfalls in oxidierender Umgebung instabil sind, sowie Teilchen mindestens eines wasserabsorbierenden Mittels dispergiert sind, wobei die Speicherschicht kompakt und wasserfrei ist.

2. Pflaster nach Anspruch 1, dadurch gekennzeichnet, daß die hydrophobe Polymermatrix auf einem Silikonpolymer oder einem Polyurethan basiert.

3. Pflaster nach Anspruch 1 oder 2, dadurch gekennzeichnet, dass es selbstklebend ist.

4. Pflaster nach Anspruch 1 oder 2, dadurch gekennzeichnet, dass es nicht klebend ist.

5. Pflaster nach einem der vorstehenden Ansprüche, dadurch gekennzeichnet, dass die Polymermatrix auf Basis eines Silikonpolymers erhalten wird durch Vernetzung eines linearen Organopolysiloxans, das am Siliziumatom mit Gruppen substituiert ist, ausgewählt unter einer C₁-C₆-Alkylgruppe, einer Arylgruppe oder einer Ar-(C₁-C₂-alkyl)-Gruppe, wobei die endständigen Siliziumatome dreifach substituiert sind.

6. Pflaster nach Anspruch 5, dadurch gekennzeichnet, dass das Organopolysiloxan folgende allgemeine Formel aufweist: 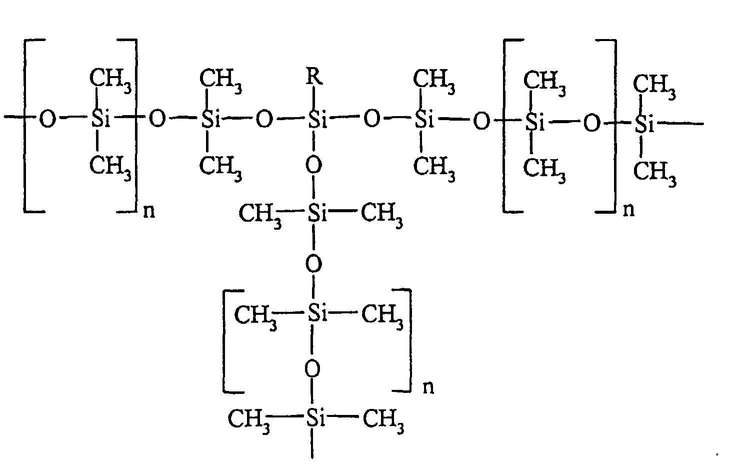 worin:
R eine Alkyl- oder Alkoxygruppe mit 1 bis 7 Kohlenstoffatomen, eine Vinylgruppe oder eine Phenylgruppe ist, und in der n etwa 100 bis 5000 beträgt.

7. Pflaster nach einem der Ansprüche 1 bis 4, dadurch gekennzeichnet, daß die Polymermatrix auf Basis eines Polyurethans durch Polyaddition eines Polyesterpolyols oder eines Polyetherpolyols in Anwesenheit eines Isocyanats oder Polyisocyanats, ausgewählt unter Toluoldiisocyanat, Diphenylmethan-4,4'-diisocyanat, Naphthalin-1,5-diisocyanat und Isophorondiisocyanat, erhalten wird.

8. Pflaster nach einem der vorstehenden Ansprüche, dadurch gekennzeichnet, dass die kosmetisch oder dermo-pharmazeutisch aktive Verbindung ausgewählt wird unter Vitamin C, Vitamin A, Vitamin F, Enzymen und Antibiotika.

9. Pflaster nach einem der vorstehenden Ansprüche, dadurch gekennzeichnet, dass das wasserabsorbierende Mittel ausgewählt wird unter superabsorbierenden vernetzten Polyacrylaten, Polyvinylalkohol, Carboxyvinylpolymeren, halbsynthetischen Cellulosederivaten, Stärken, Guargummen, Gummi arabikum oder Traganth, Casein, Phytocolloiden, Baumwollfasern und Gelatine.

10. Pflaster nach einem der Ansprüche 1 bis 8, dadurch gekennzeichnet, dass das wasserabsorbierende Mittel in Form von Teilchen eines lyophilisierten oder zerstäubten Emulsionspulvers vorliegt, das gegebenenfalls mindestens eine Wirksubstanz enthält.

11. Pflaster nach einem der vorstehenden Ansprüche, dadurch gekennzeichnet, dass der Wirkstoff in der Speicherschicht in einem Anteil von etwa 0,2 bis 48 Gew.-%, bezogen auf das Gesamtgewicht der Schicht, vorliegt.

12. Pflaster nach einem der Ansprüche 1 bis 10, dadurch gekennzeichnet, dass das wasserabsorbierende Mittel in der Speicherschicht in einem Anteil von etwa 0,1 bis 30 Gew.-%, bezogen auf das Gesamtgewicht der Schicht, vorliegt.

13. Pflaster nach den Ansprüchen 11 und 12, dadurch gekennzeichnet, dass das Gemisch des Wirkstoffes und des absorbierenden Mittels in einem Anteil von 15 bis 60 Gew. -%, bezogen auf das Gesamtgewicht der Speicherschicht, vorliegt.

14. Pflaster nach einem der vorstehenden Ansprüche, dadurch gekennzeichnet, dass die Teilchen des Wirkstoffs und des wasserabsorbierenden Mittels eine durchschnittliche Größe von etwa 0,2 µm bis 1,5 mm aufweisen.

15. Pflaster nach einem der vorstehenden Ansprüche, dadurch gekennzeichnet, dass die Speicherschicht außerdem mindestens einen kosmetischen oder dermo-pharmazeutischen Bestandteil umfaßt, ausgewählt unter weichmachenden Ölen, Pulvern von Sojaproteinen oder Getreideproteinen.

16. Pflaster nach einem der vorstehenden Ansprüche, dadurch gekennzeichnet, dass die Speicherschicht außerdem ein Schäumungsmittel umfaßt, ausgewählt unter Natriumhydrogencarbonat und Natriumcarbonat.

17. Pflaster nach einem der vorstehenden Ansprüche, dadurch gekennzeichnet, dass die Speicherschicht einen Schuß umfaßt, ausgewählt unter einem perforierten Blatt eines Plastikmaterials, einem perforierten Blatt von nicht gewebten, natürlichen oder synthetischen Fasern und einem natürlichen oder synthetischen Fasernetz.

18. Pflaster nach einem der vorstehenden Ansprüche, dadurch gekennzeichnet, dass die Dicke der Speicherschicht etwa 0,1 mm bis 12 mm beträgt.

19. Pflaster nach einem der vorstehenden Ansprüche, dadurch gekennzeichnet, dass die Trägerschicht aus einem Polymer besteht, ausgewählt unter Polyethylenen hoher und niedriger Dichte, Polypropylenen, Polyvinylchloriden, Copolymeren von Ethylen und Vinylacetat, Polyestern und Polyurethanen.

20. Pflaster nach einem der vorstehenden Ansprüche, dadurch gekennzeichnet, dass die Dicke der Trägerschicht etwa 0,2 mm bis 1,5 mm beträgt.

21. Kosmetische Verwendung eines Pflasters für die kontrollierte Freisetzung mindestens einer kosmetisch wirksamen Verbindung auf der Haut durch Anbringen des Pflasters auf deren Oberfläche, wobei das Pflaster eine kompakte und wasserfreie Speicherschicht umfaßt, die auf einer Trägerschicht angebracht ist und aus einer hydrophoben Polymermatrix besteht, in der in homogener Weise Teilchen eines Wirkstoffs, der gegebenenfalls in oxidierender Umgebung instabil ist, und Teilchen mindestens eines wasserabsorbierenden Mittels dispergiert sind, wobei eine therapeutische Verwendung ausgeschlossen wird.

22. Kosmetische Verwendung eines Pflasters gemäß Anspruch 21, dadurch gekennzeichnet, dass das Pflaster in trockenem Zustand und in verschließender Weise auf die Hautoberfläche aufgebracht wird.

23. Kosmetische Verwendung eines Pflasters gemäß Anspruch 21, dadurch gekennzeichnet, dass das Pflaster nach Befeuchtung der Hautoberfläche aufgebracht wird.
